# EUROPEAN PATENT APPLICATION

(11) **EP 4 783 177 A1**
(43) Date of publication of application: **29.07.2026**
(21) Application number: 24868257.7
(22) Date of filing: 17.09.2024
(51) Int. Cl.: G16C 20/70, G06F 16/9032, G16C 60/00

(54) **DESIGN SUPPORT DEVICE, DESIGN SUPPORT METHOD, PROGRAM, AND DESIGN SUPPORT SYSTEM**

(30) Priority: 21.09.2023 JP 2023155551
(71) Applicant: Resonac Corporation, Tokyo 105-7325 (JP)
(72) Inventor: NISHINO, Shogo, Tokyo 105-7325 (JP); SHIMIZU, Yohei, Tokyo 105-7325 (JP); OKUNO, Yoshishige, Tokyo 105-7325 (JP)
(74) Representative: Strehl & Partner mbB
(86) International application number: PCT/JP2024/033154
(87) International publication number: WO 2025/063180

(57) **Abstract**

A design support device includes: input reception unit for receiving input of first blend condition from a worker in conversational style; prompt generation unit for generating a prompt incorporating first blend condition; first search unit for searching for experimental data matching first blend condition from database storing past experimental data, using search condition generated by language model based on the prompt, and when experimental data matching first blend condition is not found, searching for experimental data matching second blend condition eased from first blend condition; prediction unit for predicting physical properties of a plurality of candidate blends generated based on a blend of experimental data matching second blend condition; second search unit for searching for candidate blend matching first blend condition from the plurality of candidate blends based on the search condition; and blend display unit for displaying the candidate blend matching first blend condition.

## Description

### TECHNICAL FIELD

The present disclosure relates to a design support device, a design support method, a program, and a design support system.

### BACKGROUND ART

In recent years, in the field of material development, artificial intelligence (AI) has been utilized to improve the efficiency of material development. By quantitatively specifying a blend from multiple options, workers engaging in material development utilizing AI have let the AI predict physical properties.

For example, in a research and development support system for improving the efficiency of the research and development, a technology using, as a personal research assistant, a research and development chatbot utilizing AI has been conventionally known (see, for example, PTL 1).

### CITATION LIST

### PATENT LITERATURE

PTL 1: Japanese Patent Application Laid-Open Publication No. 2020-52602

### SUMMARY OF THE INVENTION

### TECHNICAL PROBLEM

However, when it is necessary to quantitatively specify a blend from multiple options in order to find a desired blend, increased time and labor of the worker may be needed for inputting data, resulting in an increased workload of the worker. PTL 1 does not describe such content.

It is an object of the present disclosure to provide a design support device, a design support method, a program, and a design support system for reducing the workload of a worker who is responsible for discovering a desired blend.

### SOLUTION TO THE PROBLEM

The present disclosure includes the following configuration.
[1] A design support device, including:
   an input reception unit configured to receive an input of a first blend condition from a worker in a conversational style;
   a prompt generation unit configured to generate a prompt incorporating the first blend condition;
   a first search unit configured to search for experimental data matching the first blend condition from a database storing past experimental data, using a search condition generated by a language model based on the prompt, and when experimental data matching the first blend condition is not found, search for experimental data matching a second blend condition eased from the first blend condition;
   a prediction unit configured to predict physical properties of a plurality of candidate blends generated based on a blend of experimental data matching the second blend condition;
   a second search unit configured to search for the candidate blend matching the first blend condition from the plurality of candidate blends based on the search condition; and
   a blend display unit configured to display the candidate blend matching the first blend condition.
[2] The design support device according to [1],
   wherein when the candidate blend matching the first blend condition is not found, the second search unit searches for the candidate blend matching the second blend condition eased from the first blend condition, and
   wherein the blend display unit displays the candidate blend matching the second blend condition.
[3] The design support device according to [1] or [2], further including:
   a search condition generation unit configured to generate the search condition by the language model based on the prompt.
[4] The design support device according to [3],
   wherein the input reception unit receives an input of the first blend condition which is non-quantitative from the worker in the conversational style, and
   wherein the search condition generation unit generates the search condition by the language model based on the prompt incorporating the first blend condition which is non-quantitative received from the worker.
[5] The design support device according to any one of [1] to [4],
   wherein the prediction unit predicts the physical properties of the candidate blends using a machine learning model that has been trained about correspondence between the blends and the physical properties.
[6] The design support device according to any one of [1] to [5],
   wherein when experimental data matching the first blend condition is found, the blend display unit displays the experimental data matching the first blend condition.
[7] A design support method, including causing a computer to execute:
   an input reception step of receiving an input of a first blend condition from a worker in a conversational style;
   a prompt generation step of generating a prompt incorporating the first blend condition;
   a first search step of searching for experimental data matching the first blend condition from a database storing past experimental data, using a search condition generated by a language model based on the prompt, and when experimental data matching the first blend condition is not found, searching for experimental data matching a second blend condition eased from the first blend condition;
   a prediction step of predicting physical properties of a plurality of candidate blends generated based on a blend of experimental data matching the second blend condition;
   a second search step of searching for the candidate blend matching the first blend condition from the plurality of candidate blends based on the search condition; and
   a blend display step of displaying the candidate blend matching the first blend condition.
[8] A program for causing a computer to execute:
   an input reception procedure for receiving an input of a first blend condition from a worker in a conversational style;
   a prompt generation procedure for generating a prompt incorporating the first blend condition;
   a first search procedure for searching for experimental data matching the first blend condition from a database storing past experimental data, using a search condition generated by a language model based on the prompt, and when experimental data matching the first blend condition is not found, searching for experimental data matching a second blend condition eased from the first blend condition;
   a prediction procedure for predicting physical properties of a plurality of candidate blends generated based on a blend of experimental data matching the second blend condition;
   a second search procedure for searching for the candidate blend matching the first blend condition from the plurality of candidate blends based on the search condition; and
   a blend display procedure for displaying the candidate blend matching the first blend condition.
[9] A design support system, including a plurality of computers, the design support system including:
   an input reception unit configured to receive an input of a first blend condition from a worker in a conversational style;
   a prompt generation unit configured to generate a prompt incorporating the first blend condition;
   a first search unit configured to search for experimental data matching the first blend condition from a database storing past experimental data, using a search condition generated by a language model based on the prompt, and when experimental data matching the first blend condition is not found, search for experimental data matching a second blend condition eased from the first blend condition;
   a prediction unit configured to predict physical properties of a plurality of candidate blends generated based on a blend of experimental data matching the second blend condition;
   a second search unit configured to search for the candidate blend matching the first blend condition from the plurality of candidate blends based on the search condition; and
   a blend display unit configured to display the candidate blend matching the first blend condition.

### ADVANTAGEOUS EFFECTS OF THE INVENTION

According to the present disclosure, it is possible to provide a design support device, a design support method, a program, and a design support system for reducing the workload of a worker responsible for discovering a desired blend.

### BRIEF DESCRIPTION OF THE DRAWINGS

[FIG. 1] FIG. 1 is a configuration diagram of an example of a design support system according to an embodiment.
[FIG. 2] FIG. 2 is a hardware configuration diagram of an example of a computer according to the present embodiment.
[FIG. 3] FIG. 3 is a functional configuration diagram of an example of a design support system according to the present embodiment.
[FIG. 4] FIG. 4 is a flowchart showing an example of a process of the design support system according to the present embodiment.
[FIG. 5] FIG. 5 is an image diagram of an example of a screen for receiving an input of a first blend condition in a conversational style.
[FIG. 6] FIG. 6 is an image diagram of an example of a screen for receiving an input of a first blend condition in a conversational style.
[FIG. 7] FIG. 7 is an explanatory diagram of an example of a prompt incorporating a first blend condition.
[FIG. 8] FIG. 8 is an explanatory diagram of an example of a search condition for searching for experimental data matching the first blend condition from past experimental data.
[FIG. 9] FIG. 9 is an image diagram of an example of a screen displaying experimental data matching a second blend condition eased from the first blend condition.
[FIG. 10] FIG. 10 is an explanatory diagram of an example of blending generation conditions.
[FIG. 11] FIG. 11 is an image diagram of an example of a screen displaying candidate blends matching the first blend condition.
[FIG. 12] FIG. 12 is an explanatory diagram of an example of a screen for inputting blend conditions.
[FIG. 13] FIG. 13 is an explanatory diagram of an example of a large language model used in the design support system according to the present embodiment.

### DETAILED DESCRIPTION OF THE INVENTION

Next, embodiments of the present invention will be described in detail. The present invention is not limited to the following embodiments.

### [First Embodiment]

### <System Configuration>

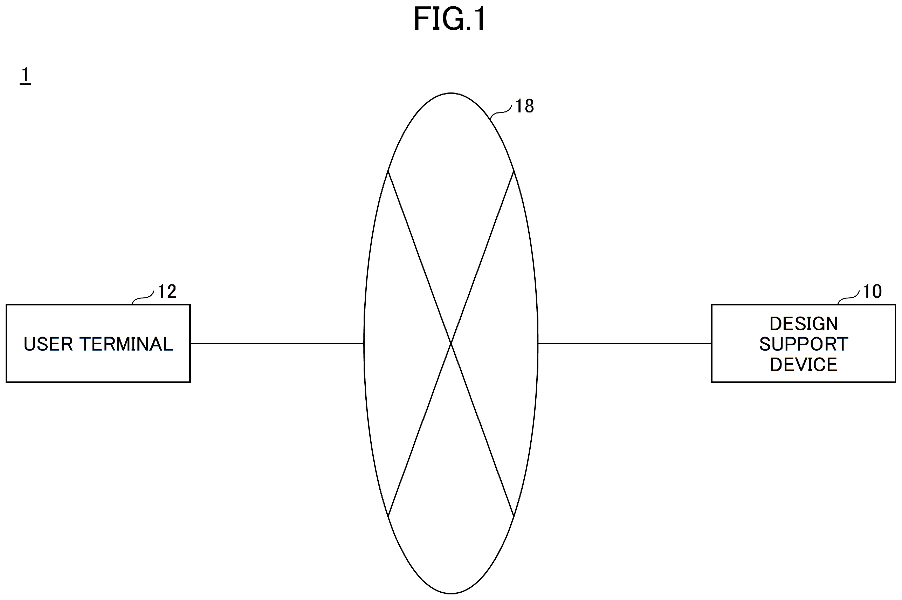

FIG. 1 is a configuration diagram of an example of a design support system according to the present embodiment. A design support system 1 of FIG. 1 includes a design support device 10 and a user terminal 12. The design support device 10 and the user terminal 12 are connected to each other via a communication network 18, such as a local area network (LAN), the Internet, and the like, so as to enable data communication.

The user terminal 12 is an information processing terminal, such as a PC, a tablet terminal, a smartphone, and the like, operated by a worker. The user terminal 12 displays a screen for receiving an input of information from the worker on a display device, and receives an input of information from the worker. The user terminal 12 transmits the information received from the worker to the design support device 10 to cause the design support device 10 to execute processing for reducing the workload of the worker responsible for discovering a desired blend.

The user terminal 12 receives information about a process execution result of the design support device 10, and displays it on the display device, for the worker to confirm it. For example, the user terminal 12 receives information about a blend desired by the worker, and displays it on the display device, for the worker to confirm it.

The design support device 10 is an information processing device, such as a PC and the like, that supports the work of the worker responsible for discovering a desired blend. The design support device 10 executes a process for reducing the workload of the worker responsible for discovering a desired blend. The design support device 10 transmits information about a process execution result, to cause the user terminal 12 to display the information about the process execution result.

For example, in the design support device 10, a language model interprets a blend condition (hereinafter referred to as a first blend condition) input from the worker in a conversational style, and searches the database for past experimental data matching the first blend condition.

When past experimental data matching the first blend condition is found, the design support device 10 causes the user terminal 12 to display it. When past experimental data matching the first blend condition is not found, the design support device 10 searches for, as a reference point, past experimental data matching a blend condition eased from the first blend condition (hereinafter referred to as a second blend condition), using the language model.

The design support device 10 generates a candidate blend close to the blend of the past experimental data matching the second blend condition, based on the blend of the experimental data, and predicts a physical property of the candidate blend, using a machine learning model that has been trained about correspondence between blends and physical properties.

When a candidate blend matching the first blend condition is found, the design support device 10 causes the user terminal 12 to display the candidate blend matching the first blend condition. When a candidate blend matching the first blend condition is not found, the design support device 10 causes the user terminal 12 to display the candidate blend matching the second blend condition.

The design support device 10 utilizes a database storing past experimental data. The design support device 10 may utilize a database stored in the design support device 10 itself, or may utilize a database, such as a database server connected via the communication network 18, and the like.

The machine training method for the machine learning model that has been machine-trained about a correspondence relationship between blends and physical properties (a trained machine learning model) is a linear method, a generalized linear method, a partial least squares method, a kernel ridge method, a Gaussian process, a k-nearest neighbor algorithm, a decision tree method, a random forest method, an adaptive boosting method, a bagging method, a gradient boosting method, a support-vector machine method, a neural network method, and the like. When predicting a physical property of a candidate blend by the trained machine learning model, a quantitative candidate blend is input into the trained machine learning model, to cause the trained machine learning model to output a physical property of the candidate blend.

The design support device 10 may use a large language model, such as ChatGPT (registered trademark) and the like, as a language model. The design support device 10 may use a language model stored in the design support device 10 itself or a language model of a server (including a cloud service) connected via the communication network 18.

The design support system 1 of FIG. 1 may be realized by the design support device 10 having a Web server function and the user terminal 12 configured to execute a Web application by a Web browser function. The design support system 1 of FIG. 1 may be realized by an application installed in the user terminal 12 executing a process in cooperation with a program installed in the design support device 10.

The design support system 1 of FIG. 1 is an example. Needless to say, there are various system configuration examples according to applications and purposes. For example, the design support device 10 may be realized by a plurality of computers, may be realized as a cloud computing service, or may be realized in cooperation with a cloud computing service. Further, the design support system 1 of FIG. 1 may be realized by a stand-alone computer.

### <Hardware Configuration>

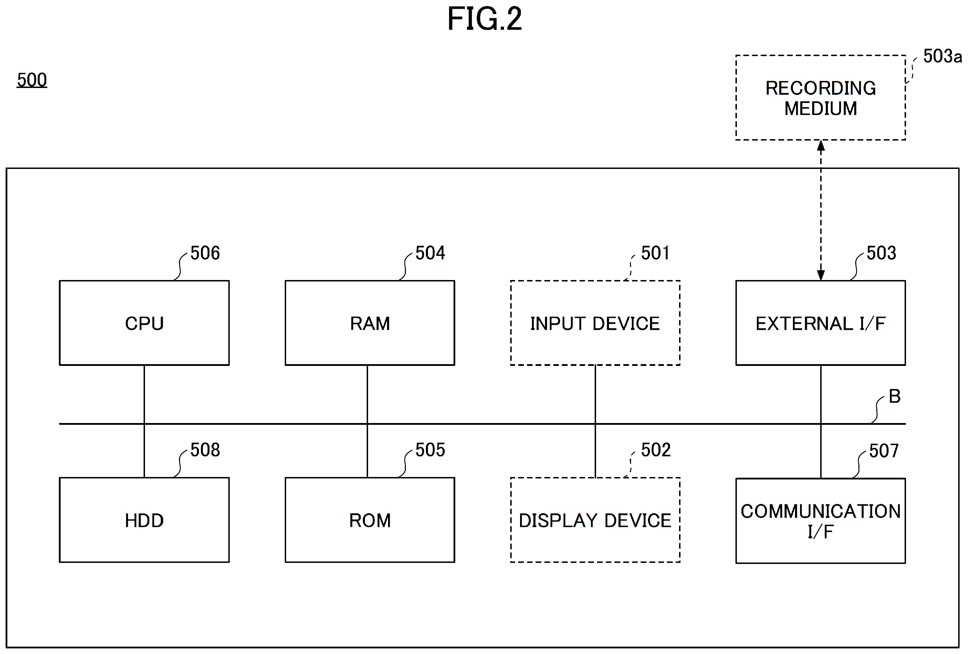

The design support device 10 and the user terminal 12 of FIG. 1 are realized by, for example, a computer 500 having the hardware configuration shown in FIG. 2.

FIG. 2 is a hardware configuration diagram of an example of a computer according to the present embodiment. The computer 500 of FIG. 2 includes an input device 501, a display device 502, an external I/F 503, a RAM 504, a ROM 505, a CPU 506, a communication I/F 507, an HDD 508, and the like, which are mutually connected via a bus B. The input device 501 and the display device 502 may be types used by being connected.

The input device 501 includes a touch panel, operation keys and buttons, a keyboard and a mouse, and the like, which are used by the worker to input various signals. The display device 502 may be composed of a liquid crystal, organic EL, or such other display for displaying a screen, and a loudspeaker and the like for outputting sound data, such as speech, sound, and the like. The communication I/F 507 is an interface for the computer 500 to perform data communication.

The HDD 508 is an example of a nonvolatile storage device for storing programs and data. The stored programs and data include an OS, which is basic software for controlling the entire computer 500, applications providing various functions on the OS, and the like. Instead of the HDD 508, the computer 500 may use a drive device (for example, a solid-state drive: SSD) using a flash memory as a storage medium.

The external I/F 503 is an interface with an external device. Examples of the external device include a recording medium 503a and the like. Thus, the computer 500 can read out from and/or write into the recording medium 503a via the external I/F 503. Examples of the recording medium 503a include a flexible disk, a CD, a DVD, an SD memory card, a USB memory, and the like.

The ROM 505 is an example of a nonvolatile semiconductor memory (storage device) that can retain programs and data even when the power is turned off. The ROM 505 stores programs and data, such as BIOS, OS settings, network settings, and the like that are executed when the computer 500 is started. The RAM 504 is an example of a volatile semiconductor memory (storage device) that temporarily retains programs and data.

The CPU 506 is an arithmetic unit that reads out programs and data from storage devices, such as the ROM 505, the HDD 508, and the like, into the RAM 504, and executes processes, thereby realizing control and functions of the entire computer 500. The computer 500 according to the present embodiment can realize various functions of the design support device 10 and the user terminal 12 as described later, by executing programs.

### <Functional Configuration>

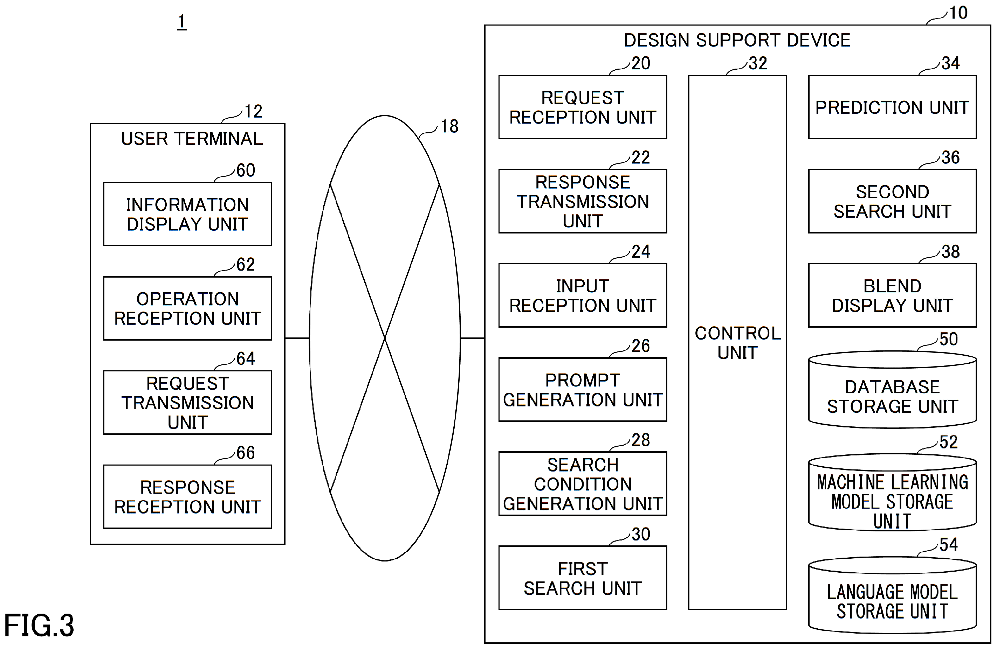

The functional configuration of the design support system 1 according to the present embodiment will be described. FIG. 3 is a functional configuration diagram of an example of the design support system according to the present embodiment. In the configuration diagram of FIG. 3, parts unnecessary for the description of the present embodiment are appropriately omitted.

The design support device 10 includes a request reception unit 20, a response transmission unit 22, an input reception unit 24, a prompt generation unit 26, a search condition generation unit 28, a first search unit 30, a control unit 32, a prediction unit 34, a second search unit 36, a blend display unit 38, a database storage unit 50, a machine learning model storage unit 52, and a language model storage unit 54. The user terminal 12 includes an information display unit 60, an operation reception unit 62, a request transmission unit 64, and a response reception unit 66.

The information display unit 60 displays, on the display device 502, a screen for receiving an input of information from a worker, and information about a process execution result of the design support device 10. The operation reception unit 62 receives an operation of a worker, such as input of information, and the like. The request transmission unit 64 transmits a request for a process corresponding to the input of information from the worker to the design support device 10. The response reception unit 66 receives from the design support device 10, a response to the request for the process transmitted by the request transmission unit 64.

The request reception unit 20 receives a request for a process from the user terminal 12. The response transmission unit 22 provides a process execution result corresponding to the request for the process, as a reply. The input reception unit 24 receives an input of a first blend condition desired by the worker in a conversation style in cooperation with the user terminal 12.

The prompt generation unit 26 generates a prompt by incorporating the first blend condition received in the conversation style into a variable part of a template prompt. Details of a prompt generated by the prompt generation unit 26 will be described later.

The search condition generation unit 28 acquires a search condition generated (output from the language model) by inputting the prompt generated by the prompt generation unit 26 to the language model. The language model interprets the first blend condition incorporated in the prompt and generates a search condition (search query) for searching for experimental data matching the first blend condition from past experimental data.

Further, the search condition generation unit 28 interprets the first blend condition incorporated in the prompt and generates a search condition for searching for experimental data matching the second blend condition eased from the first blend condition from past experimental data.

The first search unit 30 searches for experimental data matching the first blend condition from the database storing past experimental data under the search condition for searching for experimental data matching the first blend condition from past experimental data. When there is experimental data matching the first blend condition in the database storing past experimental data, the design support device 10 terminates searching for the blend desired by the worker.

When there is no experimental data matching the first blend condition in the database storing past experimental data, the first search unit 30 searches for experimental data matching the second blend condition from the database storing past experimental data under the search condition for searching for experimental data matching the second blend condition from past experimental data.

The prediction unit 34 generates a plurality of candidate blends close to the blend of the experimental data matching the second blend condition, and predicts the physical properties of the plurality of candidate blends using, for example, the trained machine learning model stored in the machine learning model storage unit 52.

The second search unit 36 refers to the predicted physical properties of the plurality of candidate blends, and searches for a candidate blend matching the first blend condition from the plurality of candidate blends. When no candidate blend matching the first blend condition is found, the second search unit 36 searches for a candidate blend matching the second blend condition from the plurality of candidate blends.

The blend display unit 38 causes the user terminal 12 to display experimental data matching the first blend condition, when there is the experimental data matching the first blend condition in the database storing past experimental data, to thereby present it to the worker.

The blend display unit 38 causes the user terminal 12 to display a candidate blend matching the first blend condition or a candidate blend matching the second blend condition when there is no experimental data matching the first blend condition in the database storing past experimental data, to thereby present the candidate blend to the worker.

The database storage unit 50 stores past experimental data. The machine learning model storage unit 52 stores the machine learning model that has been trained about correspondence between blends and physical properties. The language model storage unit 54 stores the language model for generating a search condition based on an input prompt.

The control unit 32 controls the request reception unit 20, the response transmission unit 22, the input reception unit 24, the prompt generation unit 26, the search condition generation unit 28, the first search unit 30, the prediction unit 34, the second search unit 36, the blend display unit 38, the database storage unit 50, the machine learning model storage unit 52, and the language model storage unit 54 shown in FIG. 3.

The configuration of the design support system 1 shown in FIG. 3 is an example. The design support system 1 according to the present embodiment can be realized by various configurations. For example, the database storage unit 50, the machine learning model storage unit 52, and the language model storage unit 54 may be included in a storage device, a computer, a cloud storage, and the like capable of performing data communication with the design support device 10 via the communication network 18.

### <Process>

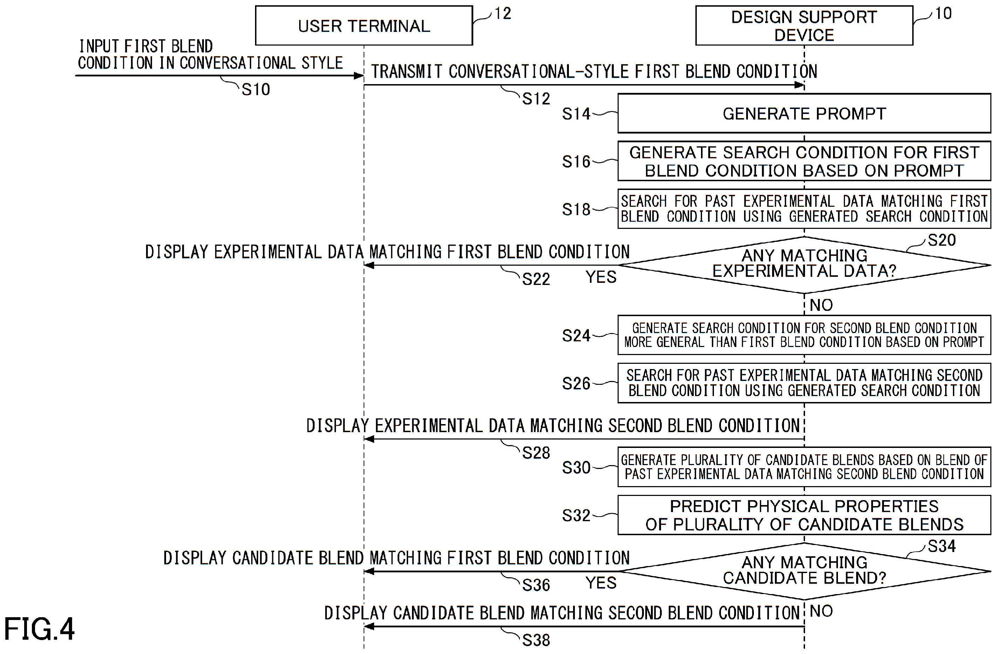

The design support system 1 according to the present embodiment supports the work of a worker responsible for discovering a blend desired by the worker, by, for example, the procedure shown in FIG. 4. FIG. 4 is a flowchart showing an example of the process of the design support system according to the present embodiment.

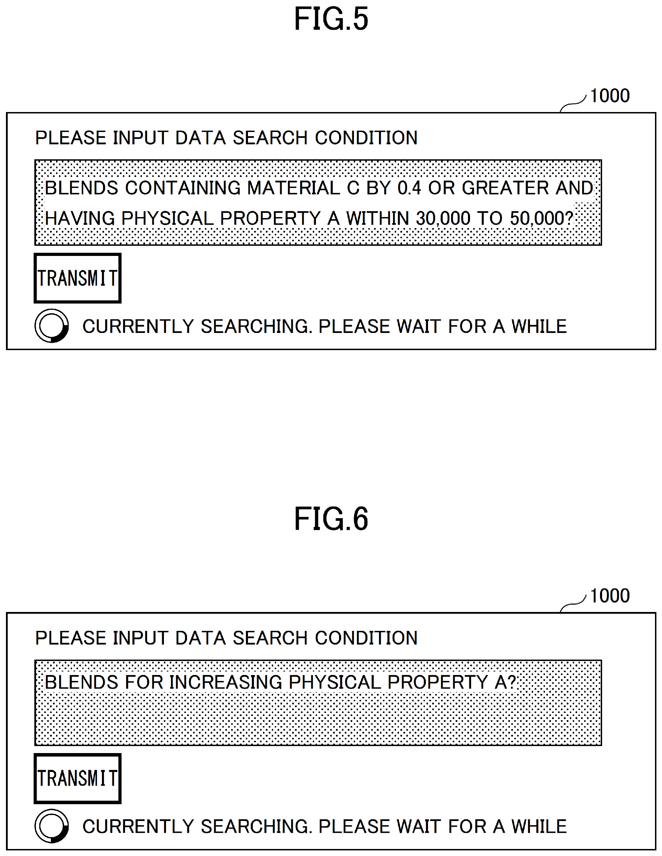

In step S10, the input reception unit 24 of the design support device 10 causes the user terminal 12 to display, for example, a screen 1000 shown in FIGS. 5 and 6. FIGS. 5 and 6 are image views of examples of a screen for receiving an input of the first blend condition in a conversational style. FIG. 5 is an example of the screen 1000 that has received from the worker, a conversational-style designation "Blends containing a material C by 0.4 or greater and having a physical property A within 30,000 to 50,000?" as the first blend condition. FIG. 6 is an example of the screen 1000 that has received from the worker, a conversational-style designation "Blends for increasing the physical property A?" as the first blend condition. As shown in FIG. 6, the worker can designate a non-quantitative first blend condition in a conversational style.

In step S12, when the user terminal 12 receives an operation of the worker to press a "Transmit" button on the screen 1000, the user terminal 12 transmits to the design support device 10, the first blend condition in the conversational style input into the screen 1000. The input reception unit 24 of the design support device 10 receives the first blend condition in the conversational style.

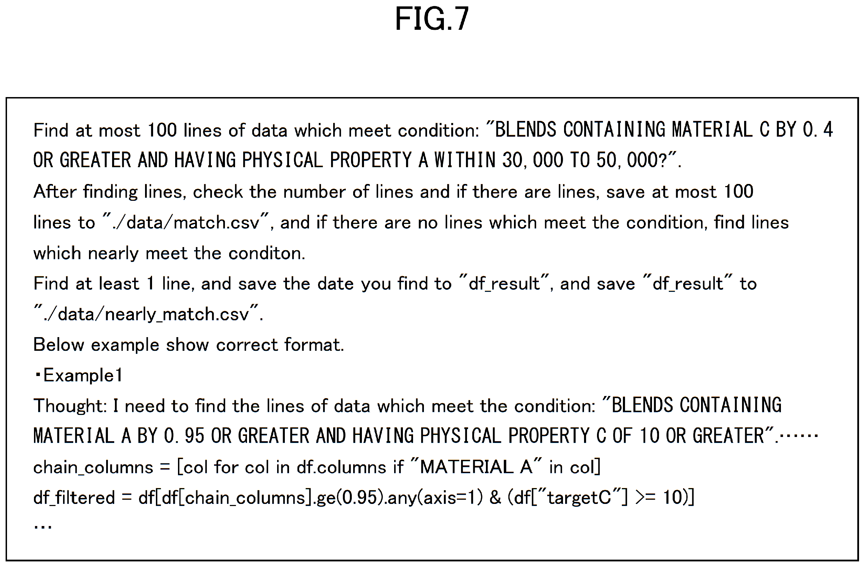

In step S14, the prompt generation unit 26 generates, for example, a prompt as shown in FIG. 7 by incorporating the conversational-style first blend condition received from the user terminal 12 into the variable part of the template prompt.

FIG. 7 is an explanatory view of an example of a prompt incorporating the first blend condition. The prompt shown in FIG. 7 has incorporated the conversational-style first blend condition "Blends containing a material C by 0.4 or greater and having a physical property A within 30,000 to 50,000?" designated by the worker via the screen 1000 of FIG. 5. Although the prompt shown in FIG. 7 is written in English to save the token count, the prompt may be written in a language other than English, such as Japanese and the like.

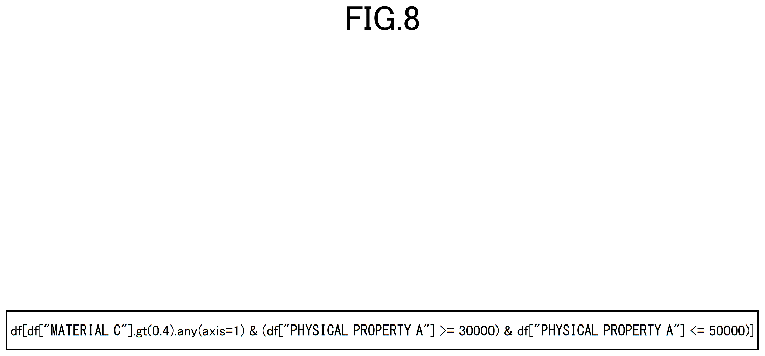

In step S16, the search condition generation unit 28 generates a first search condition by inputting the prompt generated by the prompt generation unit 26 into the language model. The language model interprets the first blend condition incorporated in the prompt and generates, for example, a search condition shown in FIG. 8 for searching for experimental data matching the first blend condition from past experimental data.

FIG. 8 is an explanatory view of an example of a search condition for searching for experimental data matching the first blend condition from past experimental data. The search condition in FIG. 8 is an example of a search condition for searching for experimental data matching the first blend condition "Blends containing a material C by 0.4 or greater and having a physical property A within 30,000 to 50,000?".

In step S18, the first search unit 30 searches for experimental data matching the first blend condition from past experimental data, using the search condition generated by the search condition generation unit 28 in step S16.

When past experimental data matching the first blend condition is found in step S20, the first search unit 30 proceeds to step S22. In step S22, the first search unit 30 notifies the blend display unit 38 of the experimental data matching the first blend condition. The blend display unit 38 causes the user terminal 12 to display the experimental data matching the first blend condition, to thereby present it to the worker.

When no past experimental data matching the first blend condition is found in step S20, the search condition generation unit 28 proceeds to step S24. In step S24, the search condition generation unit 28 interprets the first blend condition incorporated in the prompt and generates a search condition for searching for experimental data matching the second blend condition eased from the first blend condition from past experimental data. The search condition generation unit 28 may generate the second search condition by inputting into the language model, a prompt for causing the language model to generate a search condition for searching for experimental data matching the second blend condition eased from the first blend condition from past experimental data.

In step S26, the first search unit 30 searches for experimental data matching the second blend condition from past experimental data, using the search condition generated by the search condition generation unit 28 in step S24.

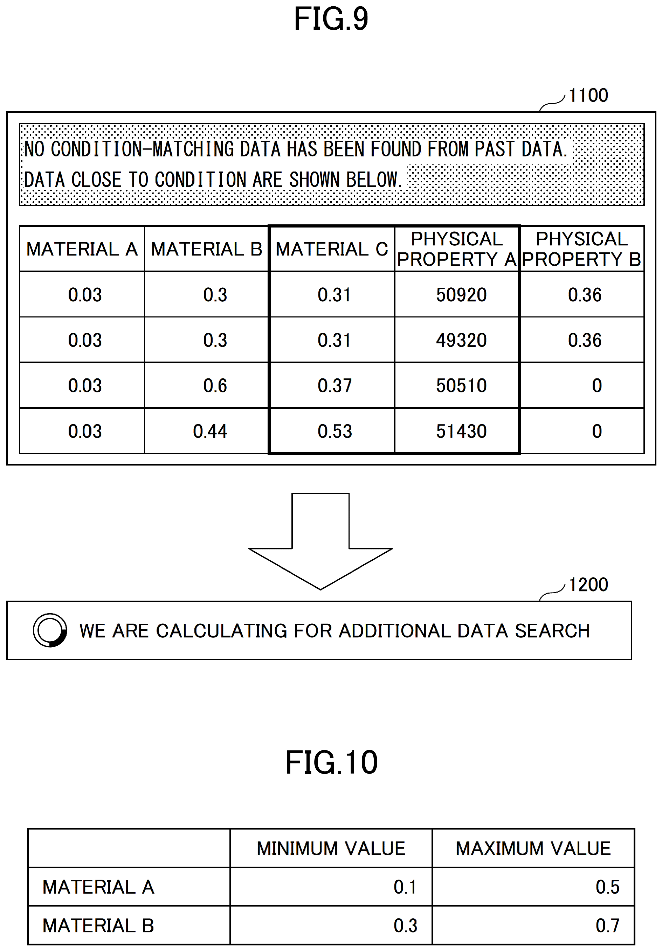

In step S28, the first search unit 30 notifies the blend display unit 38 of the experimental data matching the second blend condition. The blend display unit 38 causes the user terminal 12 to display the experimental data matching the second blend condition as shown in, for example, a screen 1100 of FIG. 9, to thereby present the data to the worker.

FIG. 9 is an image view of an example of a screen displaying the experimental data matching the second blend condition eased from the first blend condition. The screen 1100 of FIG. 9 displays past experimental data that do not match the first blend condition "Blends containing a material C by 0.4 or greater and having a physical property A within 30,000 to 50,000" but that are close to the first blend condition. Thereafter, the blend display unit 38 displays a message 1200 of FIG. 9.

In step S30, the prediction unit 34 generates a plurality of candidate blends that are close to the blends of the experimental data matching the second blend condition, using, for example, a blend generation condition of FIG. 10. FIG. 10 is an explanatory diagram of an example of the blend generation condition. The blend generation condition shown in FIG. 10 can be generated by using the past experimental data matching the second blend condition. For example, when the blending amount of the "material A" in the past experimental data matching the second blend condition is in the range of "0.1 to 0.5", the blending amount of the "material A" can be set to a random number in the range of "0.1 to 0.5".

In step S32, the prediction unit 34 predicts the physical properties of the plurality of candidate blends by using, for example, the trained machine learning model stored in the machine learning model storage unit 52.

In step S34, the second search unit 36 searches for a candidate blend matching the first blend condition from the plurality of candidate blends, using the search condition generated by the search condition generation unit 28 in step S16.

When a candidate blend matching the first blend condition is not found, the second search unit 36 searches for a candidate blend matching the second blend condition from the plurality of candidate blends, using the search condition generated by the search condition generation unit 28 in step S24.

When any candidate blend matching the first blend condition is found, the second search unit 36 proceeds to step S36. In step S36, the second search unit 36 notifies the blend display unit 38 of the candidate blend matching the first blend condition.

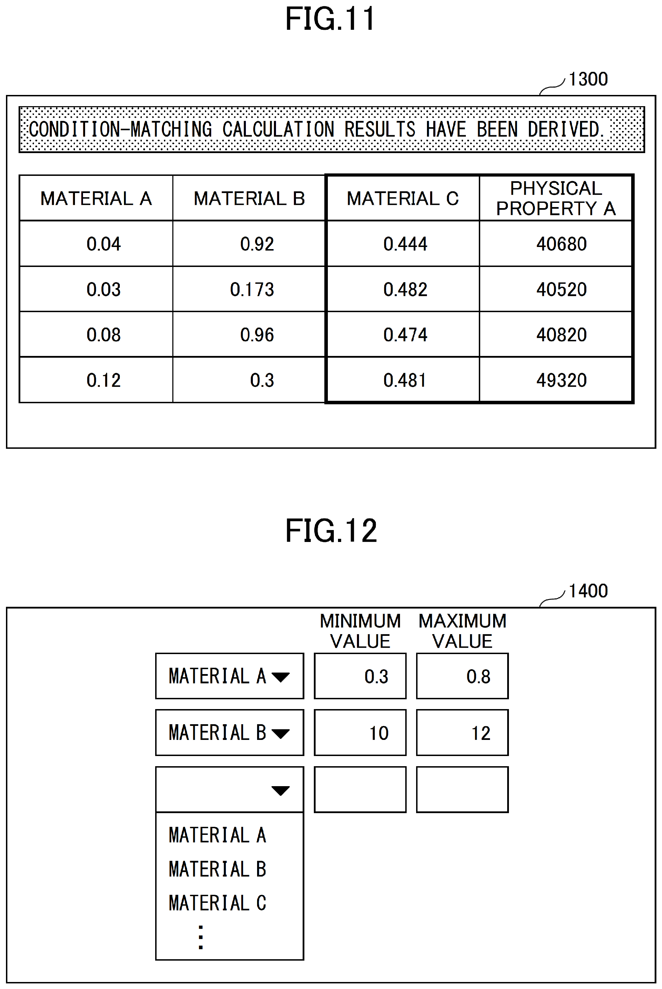

The blend display unit 38 causes the user terminal 12 to display candidate blends matching the first blend condition as shown in, for example, a screen 1300 of FIG. 11, to thereby present them to the worker. FIG. 11 is an image view of an example of the screen displaying candidate blends matching the first blend condition. The screen 1300 of FIG. 11 displays a plurality of candidate blends matching the first blend condition "blends containing a material C by 0.4 or greater and having a physical property A within 30,000 to 50,000".

When a candidate blend matching the first blend condition is not found, the second search unit 36 proceeds to step S38. In step S38, the second search unit 36 notifies the blend display unit 38 of a candidate blend matching the second blend condition. The blend display unit 38 causes the user terminal 12 to display the candidate blend matching the second blend condition, to thereby present it to the worker.

According to the process of the flowchart shown in FIG. 4, by the worker inputting the first blend condition in a conversational style, although the first blend condition is not always a quantitative one, the language model can generate an appropriate search condition and find a desired blend from past experimental data. In addition, according to the process of the flowchart shown in FIG. 4, the workload is reduced and the usability is improved more than when the blend condition is inputted as shown in a screen 1400 of FIG. 12.

FIG. 12 is an image diagram of an example of a screen for inputting the blend condition. In the screen 1400 shown in FIG. 12, the worker has to select a material from multiple options and designate the blending amount of each material quantitatively by a minimum value and a maximum value, so the workload of the worker is large. In particular, for experiments using many materials, a lot of inputting is required, which increases the workload of the worker.

In addition, according to the process of the flowchart shown in FIG. 4, by inputting the first blend condition in a conversational style, the user achieves increased chances of discovering a candidate blend that matches the first blend condition, when there is no desired blend in the past experimental data. Therefore, the worker has increased chances of discovering an unexpectedly favorable candidate blend.

In addition, according to the process of the flowchart shown in FIG. 4, experimental data that matches the second blend condition eased from the first blend condition is searched from past experimental data, and a plurality of candidate blends close to the blend of the experimental data that matches the second blend condition are generated by a random number, which reduces the possibility that unrealistic candidate blends will be proposed, even though the blend condition is not always a quantitative one. For example, according to the process of the flowchart shown in FIG. 4, a plurality of candidate blends close to the blend of the experimental data that matches the second blend condition are generated by a random number, which makes it likely that the blends satisfy required conditions (e.g., the total of main chains being 1, and the like), and increases the possibility that realistic candidate blends will be proposed.

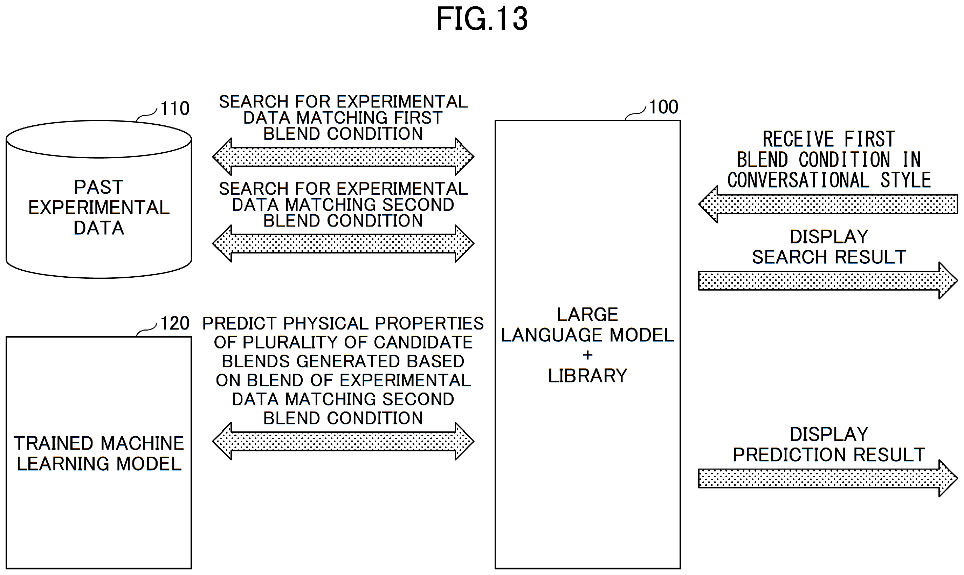

As shown in FIG. 13, for example, the design support system 1 according to the present embodiment can use a large language model 100 that is feature-expanded with a library. FIG. 13 is an explanatory diagram of an example of a large language model used in the design support system according to the present embodiment. The large language model 100 can use, for example, GPT4. As the library capable of expanding features of the large language model 100, for example, LangChain can be used.

The design support system 1 according to the present embodiment can execute, for example, a program, such as Python and the like, by using the large language model 100 that is feature-expanded with the library, and realize linkage between a search process for past experimental data 110 and a prediction process using a trained machine learning model 120 as shown in FIG. 13.

### [Other Embodiments]

For a blend proposed by the design support device 10 according to the present embodiment, materials may be synthesized by, for example, a manufacturing apparatus for synthesizing a plurality of materials, with information of the blend supplied to the apparatus.

As described above, according to the design support system 1 according to the present embodiment, it is possible to provide a design support device, a design support method, a program, and a design support system that reduce the workload of a worker responsible for searching for a desired blend.

Although the present embodiment has been described above, it will be understood that various modifications in form and particulars are applicable without departing from the spirit and scope of the claims. Although the present invention has been described based on the embodiments, the present invention is not limited to the above embodiments, and various modifications are applicable within the scope of the claims. The present application claims priority to Japanese Patent Application No. 2023-155551, filed on September 21, 2023, with the Japan Patent Office, the entire contents of which are hereby incorporated by reference.

### REFERENCE SIGNS LIST

1: design support system
10: design support device
12: user terminal
18: communication network
24: input reception unit
26: prompt generation unit
28: search condition generation unit
30: first search unit
34: prediction unit
36: second search unit
38: blend display unit

## Claims

1. A design support device, comprising:
an input reception unit configured to receive an input of a first blend condition from a worker in a conversational style;
a prompt generation unit configured to generate a prompt incorporating the first blend condition;
a first search unit configured to search for experimental data matching the first blend condition from a database storing past experimental data, using a search condition generated by a language model based on the prompt, and when experimental data matching the first blend condition is not found, search for experimental data matching a second blend condition eased from the first blend condition;
a prediction unit configured to predict physical properties of a plurality of candidate blends generated based on a blend of experimental data matching the second blend condition;
a second search unit configured to search for the candidate blend matching the first blend condition from the plurality of candidate blends based on the search condition; and
a blend display unit configured to display the candidate blend matching the first blend condition.

2. The design support device according to claim 1,
wherein when the candidate blend matching the first blend condition is not found, the second search unit searches for the candidate blend matching the second blend condition eased from the first blend condition, and
wherein the blend display unit displays the candidate blend matching the second blend condition.

3. The design support device according to claim 1 or 2, further comprising:
a search condition generation unit configured to generate the search condition by the language model based on the prompt.

4. The design support device according to claim 3,
wherein the input reception unit receives an input of the first blend condition which is non-quantitative from the worker in the conversational style, and
wherein the search condition generation unit generates the search condition by the language model based on the prompt incorporating the first blend condition which is non-quantitative, received from the worker.

5. The design support device according to any one of claims 1 to 4,
wherein the prediction unit predicts the physical properties of the candidate blends using a machine learning model that has been trained about correspondence between the blend and the physical properties.

6. The design support device according to any one of claims 1 to 5,
wherein when experimental data matching the first blend condition is found, the blend display unit displays the experimental data matching the first blend condition.

7. A design support method, comprising causing a computer to execute:
an input reception step of receiving an input of a first blend condition from a worker in a conversational style;
a prompt generation step of generating a prompt incorporating the first blend condition;
a first search step of searching for experimental data matching the first blend condition from a database storing past experimental data, using a search condition generated by a language model based on the prompt, and when experimental data matching the first blend condition is not found, searching for experimental data matching a second blend condition eased from the first blend condition;
a prediction step of predicting physical properties of a plurality of candidate blends generated based on a blend of experimental data matching the second blend condition;
a second search step of searching for the candidate blend matching the first blend condition from the plurality of candidate blends based on the search condition; and
a blend display step of displaying the candidate blend matching the first blend condition.

8. A program for causing a computer to execute:
an input reception procedure for receiving an input of a first blend condition from a worker in a conversational style;
a prompt generation procedure for generating a prompt incorporating the first blend condition;
a first search procedure for searching for experimental data matching the first blend condition from a database storing past experimental data, using a search condition generated by a language model based on the prompt, and when experimental data matching the first blend condition is not found, searching for experimental data matching a second blend condition eased from the first blend condition;
a prediction procedure for predicting physical properties of a plurality of candidate blends generated based on a blend of experimental data matching the second blend condition;
a second search procedure for searching for the candidate blend matching the first blend condition from the plurality of candidate blends based on the search condition; and
a blend display procedure for displaying the candidate blend matching the first blend condition.

9. A design support system including a plurality of computers, the design support system comprising:
an input reception unit configured to receive an input of a first blend condition from a worker in a conversational style;
a prompt generation unit configured to generate a prompt incorporating the first blend condition;
a first search unit configured to search for experimental data matching the first blend condition from a database storing past experimental data, using a search condition generated by a language model based on the prompt, and when experimental data matching the first blend condition is not found, search for experimental data matching a second blend condition eased from the first blend condition;
a prediction unit configured to predict physical properties of a plurality of candidate blends generated based on a blend of experimental data matching the second blend condition;
a second search unit configured to search for the candidate blend matching the first blend condition from the plurality of candidate blends based on the search condition; and
a blend display unit configured to display the candidate blend matching the first blend condition.
